# EUROPÄISCHE PATENTSCHRIFT

(11) **EP 1 087 759 B1**
(45) Veröffentlichungstag und Bekanntmachung des Hinweises auf die Patenterteilung: **07.08.2002**
(21) Anmeldenummer: 99927892.2
(22) Anmeldetag: 07.06.1999
(51) Int. Cl.: A61K 9/70

(54) **VERFAHREN ZUR HERSTELLUNG EINES AUS EINZELNEN SCHICHTEN BESTEHENDEN LAMINATS**
METHOD FOR MANUFACTURING A LAMINATE CONSISTING OF INDIVIDUAL LAYERS
PROCEDE DE REALISATION D'UN STRATIFIE CONSTITUE DE PLUSIEURS COUCHES INDIVIDUELLES

(30) Priorität: 15.06.1998 DE 19826592
(43) Veröffentlichungstag der Anmeldung: 04.04.2001
(73) Patentinhaber: LTS Lohmann Therapie-Systeme AG, 56626 Andernach (DE)
(72) Erfinder: VON FALKENHAUSEN, Christian, D-53125 Bonn (DE)
(74) Vertreter: Flaccus, Rolf-Dieter, Dr.
(86) Internationale Anmeldenummer: EP9903905
(87) Internationale Veröffentlichungsnummer: WO9965472

(56) Entgegenhaltungen:
- EP-A- 0 262 753
- EP-A- 0 593 807
- US-A- 4 769 028

## Beschreibung

Die Erfindung betrifft ein Verfahren zur Herstellung eines aus einzelnen Schichten bestehenden Laminats, wobei in einer Folge von Arbeitsschritten
- zuerst als Träger ein Substrat bereitgestellt, auf dieses
- eine unter Verarbeitungsbedingungen fließfähige Masse als Schicht, und auf diese Schicht
- anschließend mindestens eine weitere unter Verarbeitungsbedingungen fließfähige Masse als Schicht aufgetragen wird.

Laminate sind mehrschichtige bahnförmige Materialien. Laminate in Form von Pflastern zur Applikation auf der Haut umfassen in der Regel mindestens eine Folie und mindestens eine haftklebende Schicht. Im Sinne der Erfindung wird unter dem Begriff "Laminat" ein aus einzelnen Schichten bestehendes Gebilde verstanden, wobei fallweise keine dieser Schichten aus einer Folie besteht.
Die Herstellung mehrschichtiger Laminate erfolgt nach dem Stand der Technik üblicherweise durch Zusammenfügen einzelner einlagiger Schichten. Die Herstellung einer solchen Schicht erfolgt in der Weise, daß zunächst als Träger ein Substrat bereitgestellt wird, in der Regel in Form einer dehäsiv ausgerüsteten Folie, welche sodann mit einer "flüssigen", das heißt lösungsmittelhaltigen Polymermasse beschichtet wird. Durch anschließende Trocknung des beschichteten Substrats wird ein Abdampfen des Lösungsmittels und damit eine Vernetzung der Polymerketten in der aufgetragenen Schicht bewirkt. Die getrocknete Polymerschicht kann dann mit einer abziehbaren Folie abgedeckt werden.

Im Anschluß an die genannten Arbeitsschritte erfolgt die Herstellung eines mehrschichtigen Laminats durch Zusammenfügen mehrerer einlagiger Schichten, wobei die Deckfolie jeder Einzelschicht verworfen wird.

Im US-Patent 4,769,028 wird ein Verfahren zur Herstellung von medizinischen Verbänden beschrieben, deren wirkstoffhaltige Reservoirschicht aus einer Vielzahl von einzelnen Schichten aufgebaut ist. Diese Schichten werden nacheinander auf eine zuerst hergestellte Haftklebeschicht aufgetragen, wobei fließfähige Beschichtungsmassen, z. B. Lösungen, verwendet werden. Vor dem Auftragen der jeweils nächsten Schicht wird das in der vorher aufgetragenen Schicht noch vorhandene Löse- bzw. Dispergierungsmittel weitestgehend entfernt.

EP-A 0 593 807 offenbart ein Verfahren zur Herstellung von Pflastern zur transdermalen Verabreichung von leicht flüchtigen Wirkstoffen. Dabei werden zunächst in getrennten Arbeitsschritten aus fließfähigen Zubereitungen zwei einzelne Schichten hergestellt, wobei jeweils die Lösemittel entfernt werden. Anschließend werden die beiden Schichten zu einem Laminat vereinigt.

EP-A 0 262 753 betrifft ein transdermales System, dessen wirkstoffhaltige Matrix aus 3 unterschiedlichen Schichten aufgebaut ist. Die Herstellung erfolgt dergestalt, daß jede einzelne Schicht in einem separaten Arbeitsschritt hergestellt wird, indem eine flüssige Zubereitung in Form einer Lösung auf eine separate Folienunterlage beschichtet wird. Anschließend werden die drei einzelnen Schichten zu einem Laminat zusammengefügt.

Die bekannten Herstellungsverfahren sind in mehrfacher Hinsicht von Nachteil. Zunächst müssen in einer Folge von Arbeitsschritten einzelne Schichten hergestellt und diese danach zusammengefügt werden. Weil dabei die Abdeckfolien jeder Schicht verworfen werden, ist das Verfahren darüber hinaus materialintensiv.

Weiterhin führt die Einarbeitung flüchtiger Wirk- bzw. Hilfsstoffe in lösungsmittelhaltige Massen beim Trocknen jeder Schicht und/oder des Laminats zum Abdampfen eines unter Umständen beträchtlichen Anteils der eingearbeiteten Stoffe. Insgesamt ist das übliche Beschichtungsverfahren bei der Herstellung eines mehrschichtigen Laminats für die überwiegende Anzahl der Wirk- und Hilfsstoffe dieser Stoffklasse unwirtschaftlich und kostenintensiv, in manchen Fällen sogar ungeeignet.

Der Erfindung liegt die Aufgabe zugrunde, ein effizientes Verfahren zur Herstellung eines aus einzelnen Schichten bestehenden Laminats der im Oberbegriff von Anspruch genannten Art anzugeben, welches sich unter Vermeidung der vorgenannten Nachteile und Schwierigkeiten für eine wirtschaftlich vorteilhafte Herstellung von Laminaten mit pharmazeutischen und/oder kosmetischen Inhaltsstoffen eignet, Abdampfverluste durch Trocknung sowie Verbrauch an Deckfolienmaterial minimiert.

Die Lösung der Aufgabe gelingt mit der Erfindung bei einem Verfahren zur Herstellung eines aus einzelnen Schichten bestehenden Laminats entsprechend dem Oberbegriff von Anspruch 1 genannten Arbeitsschritten durch die im Kennzeichnungsteil von Anspruch 1 angegebene erfindungsgemäße Modifizierung des Verfahrens.

Dazu wird eine erste Masse, die sich in einem fließfähigen Zustand befindet, in Form einer ersten Schicht auf das als Träger fungierende feste Substrat aufgebracht und mindestens eine weitere Masse, die sich ebenfalls in einem fließfähigen Zustand befindet, in Form einer weiteren Schicht auf diese erste schichtförmig und im fließfähigen Zustand vorliegende erste Masse aufgebracht.

Das so hergestellte Mehrschichtlaminat kann anschließend durch äußerst vorsichtige Trocknung von Resten an Lösungsmitteln befreit werden. Auf diese Weise gehen die schichtförmig und im fließfähigen Zustand vorliegenden einzelnen Massen in einen nicht-fließfähigen, z.B. festen Zustand über. Dies kann im übrigen außer durch Trocknung auch durch chemische Vernetzung einzelner Komponenten der schichtförmigen Massen oder durch Erstarren infolge von Abkühlung einer im wesentlichen lösungsmittelfreien fließfähigen Masse erfolgen.
Als fließfähiger Zustand, was im Sinne der Erfindung mit dem Begriff "flüssig" gleichzusetzen ist, ist der Zustand gemeint, bei dem die Masse eine Konsistenz besitzt, in der sie zu einer Formänderung befähigt ist. In diesem Sinne können also insbesondere auch Schmelzen, Lösungen, Dispersionen, Suspensionen, Konglomerate etc. von z.B. makromolekularen Substanzen in einem fließfähigen Zustand vorliegen. Dieser Zustand, der die Fließfähigkeit eines Systems beschreibt, wird vom Fachmann auch durch die Angabe der Viskosität charakterisiert. Die einzelnen Massen, die in dem beschriebenen Herstellverfahren eingesetzt werden, besitzen eine dynamische Viskosität [mPa s] im Bereich von 1 bis 500 000, bevorzugt zwischen 50 und 10 000, für lösemittelhaltige Substanzen bzw. zwischen 500 und 250 000 für Schmelzen (Hotmelts), und besonders bevorzugt zwischen 500 und 5 000 für lösemittelhaltige Substanzen, bzw. zwischen 800 und 15 000 für Schmelzen (Hotmelts). Nach Abschluß des erfindungsgemäßen Verfahrens befinden sich die Massen der einzelnen Schichten in einem nicht-fließfähigen, z.B. im festen Zustand. Damit ist gemeint, daß die Massen zwar noch eine gewisse Elastizität besitzen können, aber nicht mehr zu einer dauerhaften Änderung ihrer Schichtform befähigt sind.
Das Verfahren nach der Erfindung ermöglicht die Herstellung mehrschichtiger Laminate mit einem Arbeitsschritt je Schicht unter Weglassung einer Träger- und/oder Deckfolie, wodurch Maschinenlaufzeiten und Materialkosten signifikant reduziert werden.
Durch Vermeidung einer Zwischentrocknung wird ein kostenträchtiger Einsatz von Lösungsmitteln und Trocknungsenergie zumindest erheblich reduziert und der Zeitaufwand für die Herstellung minimiert.
Durch geeignete Rezepturen der einzelnen Beschichtungsmassen lassen sich darüber hinaus unter Reduzierung der erforderlichen Fertigungsschritte beim Auftragen jeder Schicht Laminate mit speziellen Eigenschaften herstellen. Beispielsweise kann die erste Schicht einen flüchtigen Wirk- bzw. Hilfsstoff beinhalten, während die Rezeptur der folgenden Schicht wirkstofffrei bleibt. Diese wirkt während des Trocknungsvorganges für in der Vorschicht enthaltenen Wirkstoff als Sperrschicht. Somit lassen sich Laminate mit flüchtigen Wirk- und Hilfsstoffen einfacher herstellen.
Mit Vorteil kann hierfür die Maßnahme ergriffen sein, daß Schichten ohne Zwischentrocknung "flüssig auf flüssig" aufeinander aufgetragen werden. Weitere Ausgestaltungen des Verfahrens sind entsprechend den Merkmalen der Unteransprüche vorgesehen.
Eine Ausgestaltung sieht vor, daß zum Auftragen einer Schicht eine lösungsmittelhaltige Masse verwendet wird. Dabei kann auch eine Emulsion zweier miteinander nicht mischbarer Lösungsmittel verwendet werden. Es kann aber auch zum Auftragen einer Schicht eine Masse mit wenigstens einem vernetzbaren Bestandteil verwendet werden.

Weiterhin sieht eine Ausführungsform des Verfahren vor, daß das Laminat nach Aufbringen einer letzten Schicht getrocknet wird.
Dabei können für einzelne auf das Substrat aufzubringende Schichten Massen mit gleichartigen Bestandteilen verwendet werden.
Es können aber auch für den Auftrag von auf das Substrat aufzubringenden Schichten Massen mit bis auf einen Bestandteil gleichartigen Bestandteilen verwendet werden.
Im Sinne der Erfindung soll auch nicht auszuschließen sein, daß eine Schicht mit wenigstens 10 % Lösungsmittelgehalt mit einer weiteren Schicht überzogen wird.
Hierfür können Massen verwendet werden, die sich hinsichtlich ihrer Dichte und/oder Viskosität und/oder Vernetzbarkeit und/oder Feststoffgehalt und/oder Lösungsmittelgehalt und/oder pH-Wert unterscheiden.
Auch können Schichten mit unterschiedlicher Dicke aufgetragen werden, oder es können Beschichtungsbreiten einzelner Schichten gleich oder unterschiedlich breit gewählt werden. Die Erfindung schließt auch die Möglichkeit ein, daß ein einschichtiges oder mehrschichtiges Substrat als Träger des Laminats verwendet wird. Auch kann eine Mehrfachbeschichtung des Substrates mittels unterschiedlicher Auftrags- und Dosierverfahren, z.B. mittels Rakeldosierung, Walzendosierung, Fließer- bzw. Gießerdosierung, Hot-Melt-Beschichtungsverfahren oder einer Kombination aus diesen Verfahren durchgeführt werden. Schließlich kann nach dem erfindungsgemäßen Verfahren auch vorgesehen sein, daß Schichten im Druck-, Tampondruck- bzw. Siebdruckverfahren hergestellt werden.
Die Dicke der Schichten, die aus den im fließfähigen Zustand vorliegenden Massen hergestellt werden können, ist prinzipiell nicht beschränkt. Sie liegt bei dem erfindungsgemäßen Verfahren im wesentlichen zwischen 1 und 500 µm, bevorzugt zwischen 10 und 200 µm. Eine Obergrenze der Dicke der Schicht kann unter bestimmten Umständen jedoch von dem Grad der Fließfähigkeit der im fließfähigen Zustand befindlichen Massen abhängen. Bei Massen mit guter Fließfähigkeit(geringer Viskosität, also z.B. verhältnismäßig dünnflüssig; ist ohne große äußere Kraftanwendung zu einer Formänderung befähigt) kann mitunter eine solche Obergrenze der Dicke der Schicht existieren. Es ist dem Fachmann jedoch bekannt, mit welchen Maßnahmen die Fließfähigkeit der Masse beeinflußt werden kann. So führen z.B. eine Verringerung der Schmelztemperatur und/oder eine Verringerung des Lösungsmittelanteils der Lösung (oder Suspension oder Dispersion) und/oder die stärkere Vernetzung eins Polymermaterials im allgemeinen zu einer Verringerung der Fließfähigkeit. Solche Maßnahmen müssen gegebenenfalls durchgeführt werden, um die Fließfähigkeit der Massen so zu variieren, daß sie zur Herstellung von Schichten mit gewünschter Dicke verwendet werden können. Sollte jedoch eine Masse nur eine sehr schlechte Fließfähigkeit besitzen (hohe Viskosität, also z.B. verhältnismäßig zähflüssig), so ist durch Erhöhung der Schmelztemperatur und/oder eine Erhöhung des Lösungsmittelanteils der Lösung und/oder die geringere Vernetzung eines Polymermaterials und/oder durch größere äußere Kraftanwendung (z.B. durch Druck) beim Laminieren die Fließfähigkeit der Masse zu verbessern.

Das erfindungsgemäße Verfahren ist speziell geeignet zur Herstellung wirkstoffhaltiger Laminate zur topischen Applikation in Form von Pflastern, und ganz besonders hierbei im Bereich der transdermalen Medikation. Darüber hinaus lassen sich nach dem Verfahren auch wirkstoffhaltige Laminate herstellen, welche nicht zur topischen Anwendung vorgesehen sind, wie beispielsweise film- oder folienförmige Darreichungsformen.

Beschichtungsmassen einzelner Schichten können als Polymermaterial Kautschuk, kautschukähnliche Homo-, Co- oder Blockpolymere, Silikone, Polyacrylsäureester und deren Copolymerisate, Polyurethan, Copolymere des Ethylens, Polysiloxane etc. enthalten. Weiterhin können Hilfsstoffe enthalten sein, welche in üblicher Weise Funktionen als Weichmacher, Klebrigmacher, Resorptionsvermittler, Stabilisatoren oder Füllstoffe übernehmen.
Die chemische Vernetzung einer makromolekularen Substanz, d.h. eines Polymers, erfolgt mit Hilfe dem Fachmann bekannter sogenannter Vernetzer. Diese Substanzen besitzen im allgemeinen mindestens zwei funktionelle Gruppen und bewirken, daß kettenförmige Polymermoleküle im Rahmen der chemischen Vernetzung eine dreidimensionale Struktur erhalten. Es hängt von der Natur des Polymers ab, welche konkreten Substanzen hierfür in Frage kommen.

Unter erfindungsgemäß verwendeten Laminaten, beispielsweise zur Applikation auf die Haut, sind ein- oder mehrschichtige Laminate zu verstehen, deren Materialien so beschaffen sind, daß sie sich zum längerfristigen Tragen auf der Haut eignen. Dazu zählen bekannte "Tapes" wie Hansaplast®, Leukosilk®, Leukoplast® ebenso wie auch wirkstoffhaltige Pflastermaterialien wie transdermale therapeutische Systeme (TTS), die beispielsweise in K. Heilmann "Therapeutische Systeme - Konzept und Realisation programmierter Arzneiverabreichung" (4. Auflage 1984) beschrieben sind. Sie umfassen in der Regel eine Rückschicht, eine ein- oder mehrschichtige Matrix und eine abziehbare Schutzschicht. Die Rückschicht kann aus flexiblem oder nichtflexiblem Material bestehen. Substanzen, die zu ihrer Herstellung verwendet werden, sind polymere Substanzen, wie etwa Polyethylen, Polypropylen, Polyethylenterephtalat, Polyurethan oder Polyamid. Weitere infragekommende Materialien sind Polyvinylalkohol, Styrol-dien-Blockcopolymere, Polyvinylchlorid, Polymethacrylate, um nur einige Beispiele zu nennen. Auch Kombinationen der erwähnten Materialien sind einsetzbar. Als weitere Materialien können auch z.B. mit Aluminium bedampfte Folien, allein oder mit einem polymeren Substrat beschichtet, verwendet werden.

Für ablösbare Schutzfolien können im Prinzip die gleichen Materialien verwendet werden, jedoch müssen sie zusätzlich abhäsiv ausgerüstet sein. Dies kann z.B. durch Silikonisierung erreicht werden. Das Reservoir bzw. die Matrix, die ein- oder mehrschichtig ausgebildet sein können, enthalten in der Regel neben einem oder mehreren Wirkstoffen noch Hilfsstoffe als Zusätze. Infrage kommen hierfür Polymere wie Polyisobutylen, Ester des Polyvinylalkohols, Polyacrylund Polymethacrylsäureester, Naturkautschuk, Styrol-, Isopren- und Styrol-Butadien-Polymerisate, Siliconpolymere, Harzbestandteile wie gesättigte oder ungesättigte Kohlenwasserstoffharze, Derivate des Abietylalkohols und des β-Pinens, Weichmacher wie Phthalsäureester, Triglyceride und Fettsäuren. Das Polymermaterial der Matrix kann auch auf Polymeren wie Kautschuk, kautschukähnlichen synthetischen Homo-, Co- oder Blockpolymeren, Polyurethanen, Copolymeren des Ethylens oder Polysiloxanen aufgebaut sein.

Die erwähnten Zusätze - auch Hilfsstoffe genannt - werden nach ihrer Funktion in Weichmacher, Klebrigmacher, Resorptionsvermittler, Stabilisatoren oder Füllstoffe eingeteilt. Derartige Stoffe, die physiologisch unbedenklich sein müssen, sind dem Fachmann bekannt. Für die beim erfindungsgemäßen Verfahren verwendeten Laminate, insbesondere für solche, aus denen TTS hergestellt werden, können bevorzugt hautgängige pharmazeutische Wirkstoffe verwendet werden. Diese finden sich z.B. in Wirkstoffgruppen der Parasympatholytika, z.B. Scopolamin, Atropin, Benactyzin, der Cholinergika, z.B. Physostigmin, Nicotin, der Neuroleptika, z.B. Chlorpromazin, Haloperidol, der Monoaminoxidasehemmer, z.B. Tranylcypromin, Selegilin, der Sympathomimetika, z.B.

Ephedrin, D-Norpseudoephedrin, Salbutamol, Fenfluramin, der Sympatholytika und Antisympathotonika z.B. Propanolol, Timolol, Bupranolol, Clonidin, Dihydroergotamin, Naphazolin, der Anxiolytika, z.B. Diazepam, Triazolam, der Lokalanästhetika, z.B. Lidocain, der zentralen Analgetika, z.B. Fentanyl, Sufentanil, der Antirheumatika, z.B. Indomethacin, Piroxicam, Lornoxicam, der Koronartherapeutika, z.B. Glyceroltrinitrat, Isosorbiddinitrat, der Östrogene, Gestagene und Androgene, der Antihistaminika, z.B. Diphenhydramin, Clemastin, Terfenadin, der Prostaglandinderivate, der Vitamine, z.B. Vitamin E, Cholecalciferol und der Cytostatika.

Mit dem erfindungsgemäßen Verfahren der "flüssig auf flüssig"-Beschichtung von Massen mit wirk- bzw. hilfsstoffhaltigen Inhaltsstoffen, können mehrschichtige Laminate in einer geschlossenen Folge äußerst wirtschaftlicher Herstellungsprozesse erarbeitet werden. Mit großem Vorteil ist dabei speziell die Herstellung von Laminaten möglich, welche flüchtige Wirk- bzw. Hilfsstoffe enthalten. Insoweit löst die Erfindung in optimaler Weise die eingangs gestellte Aufgabe.

## Patentansprüche

1. Verfahren zur Herstellung eines aus Einzelschichten bestehenden Laminats, wobei in einer Folge von Arbeitsschritten
- zuerst als Träger ein Substrat bereitgestellt und auf dieses
- mindestens eine unter Verarbeitungsbedingungen fließfähige erste Masse als erste Schicht aufgetragen wird, und
- anschließend mindestens eine weitere, unter Verarbeitungsbedingungen fließfähige Masse als weitere Schicht aufgetragen wird, und
- wobei zumindest eine Schicht wenigstens einen pharmazeutischen oder kosmetischen Wirkstoff und/oder Hilfsstoff enthält,
und wobei das Verfahren **dadurch gekennzeichnet ist, daß** die genannten Schichten nach dem Prinzip "flüssig-auf-flüssig" aufeinander aufgetragen werden, wobei jeweils eine fließfähige Masse auf eine schichtförmig und im fließfähigen Zustand vorliegende erste Masse aufgetragen wird.

2. Verfahren nach Anspruch 1, **dadurch gekennzeichnet, daß** die Schichten ohne Zwischentrocknung aufeinander aufgetragen werden.

3. Verfahren nach Anspruch 1 oder 2, **dadurch gekennzeichnet, daß** wenigstens eine Schicht ohne Wirkstoff oder Hilfsstoff, bevorzugt als Sperrschicht, auf eine wirkstoffhaltige Schicht aufgetragen wird.

4. Verfahren nach einem oder mehreren der Ansprüche 1 bis 3, **dadurch gekennzeichnet, daß** zum Auftragen einer Schicht eine Masse mit wenigstens einem Lösungsmittel verwendet wird.

5. Verfahren nach einem oder mehreren der Ansprüche 1 bis 4, **dadurch gekennzeichnet, daß** als Beschichtungsmasse eine Emulsion zweier miteinander nicht mischbarer Lösungsmittel verwendet wird.

6. Verfahren nach einem oder mehreren der Ansprüche 1 bis 5, **dadurch gekennzeichnet, daß** eine Schicht haftklebend ist.

7. Verfahren nach einem oder mehreren der Ansprüche 1 bis 6, **dadurch gekennzeichnet, daß** zum Aufkaschieren einer Schicht eine Masse mit wenigstens einem vernetzbaren Bestandteil verwendet wird.

8. Verfahren nach einem oder mehreren der Ansprüche 1 bis 7, **dadurch gekennzeichnet, daß** das Laminat nach Aufbringen einer letzten Schicht getrocknet wird.

9. Verfahren nach einem oder mehreren der Ansprüche 1 bis 8, **dadurch gekennzeichnet, daß** für mindestens zwei auf das Substrat aufzubringende Schichten Beschichtungsmassen mit gleichartigen Bestandteilen verwendet werden.

10. Verfahren nach einem oder mehreren der Ansprüche 1 bis 9, **dadurch gekennzeichnet, daß** für mindestens zwei auf das Substrat aufzubringende Schichten Beschichtungsmassen mit bis auf einen Bestandteil gleichartigen Bestandteilen verwendet werden.

11. Verfahren nach einem der mehreren der Ansprüche 1 bis 10, **dadurch gekennzeichnet, daß** eine Schicht mit wenigstens 10 % Lösungsmittelgehalt mit einer zweiten Schicht beschichtet wird.

12. Verfahren nach einem oder mehreren der Ansprüche 1 bis 11, **dadurch gekennzeichnet, daß** Beschichtungsmassen verwendet werden, die sich hinsichtlich ihrer Dichte und/oder Viskosität, Vernetzbarkeit, Feststoffgehalt, Lösungsmittelgehalt oder pH-Wert unterscheiden.

13. Verfahren nach einem oder mehreren der Ansprüche 1 bis 12, **dadurch gekennzeichnet, daß** Schichten mit unterschiedlicher Dicke aufgetragen werden.

14. Verfahren nach einem oder mehreren der Ansprüche 1 bis 13, **dadurch gekennzeichnet, daß** Beschichtungsbreiten einzelner Schichten gleich oder unterschiedlich breit gewählt werden.

15. Verfahren nach einem oder mehreren der Ansprüche 1 bis 14, **dadurch gekennzeichnet, daß** ein einschichtiges oder mehrschichtiges Substrat verwendet wird.

16. Verfahren nach einem oder mehreren der Ansprüche 1 bis 15, **dadurch gekennzeichnet, daß** eine Mehrfachbeschichtung des Substrats mittels unterschiedlicher Auftrags- und Dosierverfahren durchgeführt wird.

17. Verfahren nach einem oder mehreren der Ansprüche 1 bis 16, **dadurch gekennzeichnet, daß** Schichten im Druck-, Tampondruck- bzw. Siebdruckverfahren oder im Hot-Melt-Auftragsverfahren hergestellt werden.

## Claims

1. A process for producing a laminate comprising individual layers, said process comprising a sequence of operations in which
- first a support substrate is provided, to which
- at least one first composition which is flowable under processing conditions is applied as a first layer, and
- subsequently at least one further composition which is flowable under processing conditions is applied as a further layer, and
- with at least one layer comprising at least one active pharmaceutical or cosmetic substance and/or pharmaceutical or cosmetic auxiliary.
and with the said process being **characterized in that** the said layers are applied to one another according to the "liquid-on-liquid" principle, with one flowable composition in each case being applied to a first composition, which first composition is in the form of a layer and is in a flowable state.

2. The process as claimed in claim 1, **characterized in that** the layers are applied to one another without intermediate drying.

3. The process as claimed in claim 1 or 2, **characterized in that** at least one layer is applied without active substance or auxiliary, preferably as a barrier layer, to a layer comprising active substance.

4. The process as claimed in one or more of claims 1 to 3, **characterized in that** a composition comprising at least one solvent is used to apply one layer.

5. The process as claimed in one or more of claims 1 to 4, **characterized in that** an emulsion of two mutually immiscible solvents is used as coating composition.

6. The process as claimed in one or more of claims 1 to 5, **characterized in that** one layer is pressure sensitively adhesive.

7. The process as claimed in one or more of claims 1 to 6, **characterized in that** a composition comprising at least one crosslinkable constituent is used to apply one layer by lamination.

8. The process as claimed in one or more of claims 1 to 7, **characterized in that** following the application of a final layer the laminate is dried.

9. The process as claimed in one or more of claims 1 to 8, **characterized in that** coating compositions comprising the same kind of constituents are used for at least two layers intended for application to the substrate.

10. The process as claimed in one or more of claims 1 to 9, **characterized in that** coating compositions comprising the same kind of constituents except for one constituent are used for at least two layers intended for application to the substrate.

11. The process as claimed in one or more of claims 1 to 10, **characterized in that** a layer with a solvent content of at least 10% is coated with a second layer.

12. The process as claimed in one or more of claims 1 to 11, **characterized in that** the coating compositions used differ with respect to their density and/or viscosity, crosslinkability, solids content, solvent content, or pH.

13. The process as claimed in one or more of claims 1 to 12, **characterized in that** layers differing in thickness are applied.

14. The process as claimed in one or more of claims 1 to 13, **characterized in that** individual layers are selected with the same or different coating widths.

15. The process as claimed in one or more of claims 1 to 14, **characterized in that** a single-layer or multilayer substrate is used.

16. The process as claimed in one or more of claims 1 to 15, **characterized in that** multiple coating of the substrate is carried out by means of different application and metering techniques.

17. The process as claimed in one or more of claims 1 to 16, **characterized in that** layers are produced by printing techniques, including pad printing and/or screen printing techniques, or by the hot-melt application technique.

## Revendications

1. Procédé pour la fabrication d'un laminat constitué de différentes couches, dans lequel, dans une succession d'étapes de travail
- on prépare d'abord comme support un substrat et on applique sur celui-ci
- au moins une première masse pouvant s'écouler sous les conditions de transformation comme première couche et
- ensuite au moins une autre masse pouvant s'écouler sous les conditions de transformation comme autre couche et
- au moins une couche contenant au moins une substance active et/ou un adjuvant pharmaceutique ou cosmétique,
le procédé étant **caractérisé en ce que** les couches mentionnées sont appliquées l'une sur l'autre selon le principe "liquide-sur-liquide", une masse pouvant s'écouler étant à chaque fois appliquée sur une première masse disposée au préalable en forme de couche et à l'état pouvant s'écouler.

2. Procédé selon la revendication 1, **caractérisé en ce que** les couches sont appliquées les unes sur les autres sans séchage intermédiaire.

3. Procédé selon la revendication 1 ou 2, **caractérisé en ce qu'**on applique au moins une couche sans substance active ou adjuvant, de préférence comme couche de blocage, sur une couche contenant une substance active.

4. Procédé selon l'une ou plusieurs des revendications 1 à 3, **caractérisé en ce qu'**on utilise pour l'application d'une couche une masse contenant au moins un solvant.

5. Procédé selon l'une ou plusieurs des revendications 1 à 4, **caractérisé en ce qu'**on utilise comme masse de revêtement une émulsion de deux solvants non miscibles l'un avec l'autre.

6. Procédé selon l'une ou plusieurs des revendications 1 à 5, **caractérisé en ce qu'**une couche est adhésive.

7. Procédé selon l'une ou plusieurs des revendications 1 à 6, **caractérisé en ce qu'**on utilise pour le collage d'une couche une masse contenant au moins un constituant réticulable.

8. Procédé selon l'une ou plusieurs des revendications 1 à 7, **caractérisé en ce que** le laminat est séché après l'application d'une dernière couche.

9. Procédé selon l'une où plusieurs des revendications 1 à 8, **caractérisé en ce qu'**on utilise, pour au moins deux couches à appliquer sur le substrat, des masses de revêtement contenant des constituants de même type.

10. Procédé selon l'une ou plusieurs des revendications 1 à 9, **caractérisé en ce qu'**on utilise, pour au moins deux couches à appliquer sur le substrat, des masses de revêtement contenant, à un constituant près, des constituants de même type.

11. Procédé selon l'une ou plusieurs des revendications 1 à 10, **caractérisé en ce qu'**une couche présentant une teneur en solvant d'au moins 10 % est revêtue par une deuxième couche.

12. Procédé selon l'une ou plusieurs des revendications 1 à 11, **caractérisé en ce qu'**on utilise des masses de revêtement qui se distinguent en ce qui concerne leur densité et/ou leur viscosité, leur aptitude à la réticulation, leur teneur en matières solides, leur teneur en solvant ou leur pH.

13. Procédé selon l'une ou plusieurs des revendications 1 à 12, **caractérisé en ce qu'**on applique des couches présentant des épaisseurs différentes.

14. Procédé selon l'une ou plusieurs des revendications 1 à 13, **caractérisé en ce que** les largeurs de revêtement des différentes couches sont choisies de manière à être identiques ou différentes.

15. Procédé selon l'une ou plusieurs des revendications 1 à 14, **caractérisé en ce qu'**on utilise un substrat à une ou à plusieurs couches.

16. Procédé selon l'une ou plusieurs des revendications 1 à 15, **caractérisé en ce qu'**un revêtement à plusieurs couches du substrat est réalisé au moyen de différents procédés d'application et de dosage.

17. Procédé selon l'une ou plusieurs des revendications 1 à 16, **caractérisé en ce que** les couches sont réalisées par un procédé de pression, de pression par un tampon, par sérigraphie ou dans un procédé d'application d'une masse fondue à chaud.
